# EUROPEAN PATENT APPLICATION

(11) **EP 1 481 665 A1**
(43) Date of publication of application: **01.12.2004**
(21) Application number: 03012417.6
(22) Date of filing: 30.05.2003
(51) Int. Cl.: A61K 9/00, A61K 9/12, A61P 11/00

(54) **Novel lipid mixtures for synthetic surfactants**

(71) Applicant: CHIESI FARMACEUTICI S.p.A., I-43100 Parma (IT)
(72) Inventor: Johansson, Jan, 43100 Parma (IT); Curstedt, Tore, 43100 Parma (IT); Robertson, Bengt, 43100 Parma (IT)
(74) Representative: Minoja, Fabrizio, Dr.

(57) **Abstract**

The present invention provides novel lipid mixtures for synthetic surfactants. In particular, the invention provides lipid mixtures containing a certain amount of polyunsaturated phospholipids to be used for the preparation of synthetic surfactants.

Said surfactants and pharmaceutical compositions thereof are useful for the treatment of surfactant deficiencies like respiratory distress syndrome (RDS).

## Description

### Background of the invention

Endogenous pulmonary surfactant reduces surface tension at the air-liquid interface of the alveolar lining, preventing the lungs from collapsing at end expiration. Surfactant deficiency is a common disorder in premature infants and causes respiratory distress syndrome (RDS), which can be effectively treated with preparations which are lipid extracts of minced mammalian lung or lung lavage. Said preparations are known as modified natural surfactant and they are mainly composed of phospholipids such as phosphatidylcholine (PC), phosphatidylethanolamine (PE) and phosphatidylglycerol (PG) and the hydrophobic surfactant proteins **B** and **C** (SP-B and SP-C).

For clarity, a list of PL cited in this patent application follows:
- phosphatidylcholine: PC,
- phosphatidylethanolamine: PE,
- phosphatidylglycerol (PG),
- 1,2-dipalmitoyl-*sn*-glycero-3-phosphoglycerol, generally known as dipalmitoyl-phosphatidylglycerol: DPPG
- 1,2-dipalmitoyl-*sn*-glycero-3-phosphocholine, generally known as dipalmitoyl-phosphatidylcholine: DPPC
- 1-palmitoyl-2-oleyl-PG: (POPG)
- 1-palmitoyl-2-oleyl-PC: (POPC)
- 1-palmitoyl-2-linoleyl-*sn*-glycero-3-phosphocholine, generally known as palmitoyl-linoeyl-phosphatidylcholine: PLPC
- 1-stearoyl-2-arachidonoyl-*sn*-glycero-3-phosphocholine, generally known as steaoryl-arachidonoyl-phosphocholine (SAPC)
- 1,2-dipalmitoyl-sn-glycero-3-phosphoethanolamine, generally known as dipalmitoyl- phosphatidylethanolamine(DPPE)
- 1,2-distearoyl-*sn*-glycero-3-phosphoethanolamine, generally known as distearoyl- phosphatidylethanolamine (DSPE)
- 1,2-dipalmitoyl-*sn*-glycero-3-phospho-L-serine, generally known as dipalmitoyl-phosphatidylserine (DPPS).

The glycerol moieties of the phospholipids are mainly esterified with long chain fatty acids (C₁₄-C₂₀) which in turn can be saturated (e.g. myristic, palmitic and stearic acid), monounsaturated (e.g. oleic acid) and polyunsaturated (e.g. linoleic and arachidonic acid).

The capability of the surfactants of lowering the surface tension as well as other parameters such as the spreading rate can be tested in vitro using several methods, for example the "captive bubble method" as described in Schurch, S., Bachofen, H. Goerke, J., Possmayer, H. (1989) "A captive bubble method reproduces the in situ behaviour of lung surfactant monolayers" *J*. *Appl. Physiol.,* 67: 2389-2396

One of the most important components of surfactant preparations is 1,2-dipalmitoyl-sn-glycero-3-phosphocholine (DPPC) as it forms a monomolecular film at the air-liquid interface during compression, probably undergoing phase transition (solidification) during surface compression, thereby stabilizing a system of alveoli with different sizes.

Surfactant preparations obtained from animal tissue present some drawbacks, like their availability in limited amounts , the complication of the production and sterilization processes and the relevant production costs: as a consequence, many efforts have been made to prepare synthetic surfactants.

According to Wilson (Expert Opin Pharmacother 2001, 2, 1479-1493), synthetic surfactants are distinguished in:
"artificial" surfactants devoid of surfactant proteins, simply consisting of mixtures of synthetic compounds, primarily phospholipids and other lipids that are formulated to mimic the lipid composition and behavior of natural surfactant; and
"reconstituted" surfactants which are artificial surfactants to which have been added surfactant proteins isolated from animals or manufactured through recombinant technology such as those described in WO 95/32992, or synthetic surfactant protein analogues such as those described in WO 89/06657, WO 92/22315 and WO 00/47623.

The development of reconstituted surfactants has to a large extent been focused on the surfactant protein analogues while the lipid composition has obtained less attention.

In the prior art, data about the lipid composition of artificial and/or reconstituted surfactants have been reported in the following documents.

Suzuki et al (Eur J Respir Dis 1986, 69, 336-345) described artificial surfactants made of low-molecular weight surfactant proteins and phospholipid mixture constituted of DPPC and 1,2-dipalmitoyl-*sn*-glycero-3-phosphoglycerol (DPPG) in the ratio 80:20 by weight. Suzuki generically reported that unsaturated phospholipids promote the surface adsorption of DPPC and other saturated lipids. However from *in vitro* experiments, better results in terms of film compressibility and minimal surface tension were obtained with a DPPC:DPPG mixture than mixtures of DPPC and unsaturated phospholipids.

In Notter et al (Clin Perinatology 1987, 14, 433-479) the effects of lipids or phospholipids such as dioleyl-PC, PE, cholesterol, short-chain saturated-PC, fatty acids and glycerides on the re-spreading properties of DPPC have been reviewed. The general results have been of an enhanced dynamic respreading compared to DPPC alone. However, this effect has typically not translated into significant improvements in overall surface activity or physiologic activity to levels equivalent to modified natural surfactant.

WO 89/06657 and WO 92/22315 in the name of Scripps are directed to polypeptide analogous of SP-B, Although in the specification it is generically reported that *the lipid portion* (of the surfactant compositions, editor's note) *is preferably about 50 to about 90, more preferably about 50 to 75, weight percent DPPC with the remainder unsaturated PC, PG, triacylglycerols, palmitic acid, sphingomyelin or a mixture thereof,* nevertheless in the examples, only a mixture of DPPC: PG 3:1 (75:25) by weight, in some cases further containing palmitic acid has been utilized.

In Cochrane et al (Science, 1991, 254, 566-568), the behaviour *in vitro* and *in vivo* of synthetic peptides deriving from the sequence of SP-B and phospholipid mixtures made of DPPC and PG 3:1, DPPC and 1-palmitoyl-2-oleyl PG (POPG) 3:1, DPPC and 1-palmitoyl-2-oleyl-PC (POPC) 3:1 has been investigated.

To these mixtures 15% w/w of palmitic acid was added.

In the patent application WO 95/32992 in the name of Byk Gulden directed to polypeptides analogues of SP-C, it is generally reported that the phospholipid portion could comprise DPPC, POPG (or PG) and a salt such as Ca or Mg or Na chloride to reduce viscosity. In the examples, a mixture of DPPC:POPG:palmitic acid 7:3:0.25 + CaCl₂ has been utilised.

Curstedt et al (9^{th} International Workshop on Surfactant Replacement, Jerusalem, May 22-25, 1994) reported on the in vitro activity of SP-C analogous recombined with the following lipid mixtures with or without CaCl₂: DPPC:POPC:dioleyl-PG (DOPG) 55:35:10; DPPC:PG 7:3; DPPC:PG:palmitic acid 68:22:9. The preparation containing DPPC:PG:palmitic acid had a faster adsorption rate and a lower equilibrium surface tension than the other lipid mixtures.

Krill et al *(Chemistry and Physics of Lipids 71, 47-59, 1994)* reached the same conclusions; in a study aimed at studying the in vitro properties of surfactant compositions made of a synthetic protein B fragment, DPPC and POPG they suggested that the presence of palmitic acid helps in establishing a stable lipid film of DPPC, capable of achieving low surface tension values.

In WO 00/47623 in the name of the applicant, directed to SP-C analogue peptides, it is generally reported that suitable lipids/phospholipids may be selected from the group consisting of PC (preferably DPPC), PG, palmitic acid, triacylglycerols, sphingomyelin. In the examples, surfactant preparations made of one of the claimed peptides, e.g. SP-C(LKS) in combination with DPPC:PG 7:3 w/w or DPPC:PG: palmitic acid 68:22:9 w/w/w have been tested in vitro.

In Palmblad et al (Biochem J 1999, 339, 381-386), the same data were reported. In the paper, it is also stated that optimal in vitro characteristics were also obtained from a preparation containing SP-C(LKS), SP-B, DPPC and PG, i.e. when palmitic acid was omitted from the lipid mixture.

Diemel et al (J Biol Chem, 2002, 277, 21179-21188) reported on the structural properties of surfactant mixtures made of SP-B and DPPC:DPPG 80:20 or DPPC:POPG 80:20 or DPPC:POPC:DPPG 60:20:20.

In all the documents of the prior art lipid compositions with a content of DPPC of about 60-80% by weight of total phospholipids have been utilized or suggested as preferable in particular for decreasing surface tension to low values.

However, synthetic surfactant compositions comprising relatively high contents of DPPC turn out to be not optimal in terms of rheological properties (e.g viscosity). This is particularly dramatic for highly concentrated lipid-peptide mixtures (higher than about 20 mg/ml) for which, in order to be effectively administered, it is often necessary to resort to tedious and time-consuming procedures such as heating and vigorous stirring.

In view of these drawbacks, it would be highly advantageous to provide lipid mixtures to be used in the preparation of synthetic surfactants which, while allowing to achieve low surface tension values at the air-liquid interface of the alveolar lining, are able to keep low the viscosity of the surfactant favouring its spreading and its biological activity.

### Summary of the invention

The present invention is directed to a lipid mixture for preparing a synthetic surfactant consisting of DPPC in an amount of less than 60%, preferably comprised between 40% and 60% by weight and the remainder portion containing at least 10% by weight of polyunsaturated phospholipids. It has indeed been found that by using lipid mixture containing a certain amount of polyunsaturated phospholipids it is possible to obtain low surface tension during film compression even if the content of DPPC is reduced to about 40 - 55%. It has been supposed that the polyunsaturated phospholipids are more easily squeezed out from the monomolecular film during compression, thus facilitating the formation of a pure DPPC monolayer. In addition, the synthetic surfactant preparations containing the lipid mixtures of the invention have low viscosity, favoring their delivery and distribution in lungs, so making them much more effective for treatment of pulmonary diseases.

*In vivo* experiments carried out in immature newborn rabbits have indeed proved that reconstituted surfactants containing one of the lipid mixture of the invention give rise to higher tidal volumes than those obtained with a surfactant containing a lipid mixture of the prior art.

### Detailed description of the invention

The characteristics of the lipid mixtures of the invention and the corresponding synthetic surfactant preparations will be described in the following detailed description.

Advantageously the lipid mixture contains less than 60%, preferably about 40% to about 60%, preferably about 45% to about 55% by weight of 1,2-dipalmitoyl-*sn*-glycero-3-phosphocholine (DPPC) and the remainder portion contains at least 5% by weight of polyunsaturated phospholipids, preferably more than 10%, more preferably more than 20%, even more preferably at least 30% on the total weight of phospholipids.

As polyunsaturated phospholipids we intend commercially available phospholipids (PL) such as phosphatidylcholine (PC), phosphatidylinositol (PI), phosphatidylethanolamine (PE), phosphatidylserine (PS), phosphatidylglycerol (PG) and sphingomyelin (SM) in which at least one of the esterifying fatty acid residues contains more than one double bond in the aliphatic chain. Typical polyunsaturated fatty acids are linoleic acid, linolenic acid and arachidonic acid.

The preferred polyunsaturated phospholipids of this kind are 1-palmitoyl-2-linoleyl-*sn*-glycero-3-phosphocholine (PLPC), 1-palmitoyl-2-arachidonoyl-sn-glycero-3-phosphocholine (PAPC) 1-stearoyl-2-arachidonoyl-*sn*-glycero-3-phosphocholine (SAPC) and phosphatidylethanolamine (PE). The polyunsaturated phospholipids are generally commercially available and can be either prepared by synthetic route or obtained from a natural source, from mammalian tissues. Phosphatidylethanolamine is one of the preferred unsaturated phospholipids and it is present in the composition in an amount of 1% to 5%, preferably of 2% to 5%, more preferably of at least 5%, even more preferably of at least 10% by weight. In particular, for the aim of the present invention a lipid composition comprising at least 15%, preferably 20%, more preferably 25% by weight of PE turned out to be effective. In one embodiment the lipid mixture comprises a liver extract, with average percent composition of about PC 50%, PE 25%, sphingomyelin 10%, acidic phospholipids 15%, added to DPPC and POPG.

Other lipids which can be present in the mixture of the invention are commercially available neutral lipids such as triacylglycerols, cholesterol and cholesterol esters and/or phospholipids such as those mentioned above in which the esterifying fatty acids are saturated and/or monounsaturated. Typical phospholipids of said class are 1,2-dipalmitoyl-*sn*-glycero-3-phosphoglycerol (DPPG), 1-palmitoyl-2-oleyl-*sn*-glycero-3-phosphoglycerol (POPG), 1,2-dioleyl-*sn*-glycero-3-phosphoglycerol (DOPG), 1-palmitoyl-2-oleyl-*sn*-glycero-3-phosphocholine (POPC), 1,2-dipalmitoyl-*sn*-glycero-3-phosphoethanolamine (DPPE), 1,2-distearoyl-sn-glycero-3-phosphoethanolamine (DSPE), 1,2-dipalmitoyl-sn-glycero-3-phospho-L-serine (DPPS). Preferably said other lipids in the mixture will be saturated or unsaturated acidic phospholipids such as phosphatidylglycerols, phosphatidylinositols and phosphatidyl-serines, 1,2-dipalmitoyl-*sn*-glycero-3-phosphoglycerol (DPPG) and 1-palmitoyl-2-oleyl-*sn*-glycero-3-phosphoglycerol (POPG) in an amount of at least 5% by weight, preferably higher than 10%.

In *in vivo* experiments, it has indeed been found that reconstituted surfactants containing an amount of acidic phospholipids higher than 10% give rise to better performances in terms of Lung Gas Volumes values than those containing a lower amount.

The lipid mixture of the invention is preferably devoid of palmitic acid.

As an example of lipid mixture of the invention DPPC:PLPC:SAPC:DPPG:POPG in the ratio 45:30:10:5:10 (by weight) and DPPC:liver PL:POPG in the ratio 50:40:10 have been used.

The lipid mixtures of the invention can be particularly useful in the preparation of a reconstituted surfactants.

In a particular embodiment the present invention concerns a reconstituted surfactant comprising one of previously described lipid mixtures in combination with a surfactant protein isolated from animals or manufactured through recombinant technology such as those described in WO 95/32992, or with synthetic analogues of the surfactant proteins SP-B or SP-C such as those described in WO 89/06657, WO 92/22315 and WO 00/47623 or with mixtures thereof.

The protein can be any surfactant protein, selected from SP-A, SP-B, SP-C, SP-D or a synthetic or recombinant analogue thereof.

The reconstituted surfactant of the invention can also comprise a polymyxin, preferably polymyxin B, as functional substitute of surfactant protein SP-B.

Preferably as a synthetic analogues of SP-C peptide of general formula (**I**) according to one-letter amino acid code will be utilised:

FₑG_{f}IPZZPVHLKR(XₐB)(X_{b}B)ₙ(X_{c}B)ₘ X_{d}GALLΩGL (**I**)

wherein:
X is an amino acid selected from the group consisting of I, L, nL (norleucine);
B is an amino acid selected from the group consisting of K, W, F, Y, ornithine;
Z is S optionally substituted with acyl groups containing 12-22 carbon atoms linked to the side chain via an ester or thio-ester bond, respectively;
Ω is an amino acid selected from the group consisting of M, I, L, nL (norleucine);
a is an integer from 1 to 19;
b is an integer from 1 to 19;
c is an integer from 1 to 21;
d is an integer from 0 to 20;
e is 0 or 1;
f is 0 or 1;
n is 0 or 1;
m is 0 or 1;
with the following provisos:
- n+m ≥ 0;
- f ≥ e,
- (XₐB)ₙ(X_{b}B)ₙ(X_{c}B)ₘX_{d} is a sequence having a maximum of 22 amino acids, preferably from 10 to 22.

Even more preferred is the use of peptides of formula (**II**)

IPSSPVHLKRLKLLLLLLLLILLLILGALLΩGL (**II**)

wherein:
Ω is an amino acid selected from the group consisting of M, I, L, nL (norleucine);
and wherein serine can optionally be acylated, for example with palmitoyl.

The hereinafter reported peptide (SP-C33), in the non-acylated form, is the most preferred of the invention,

IPSSPVHLKRLKLLLLLLLLILLLILGALLMGL (SP-C33)

Peptides of formula (I) have been reported in WO 00/47623.

The amount of the surfactant protein or of the peptide in the reconstituted surfactant will vary in the range of 0.001 to 0.2, preferably between 0.01 to 0.1 by weight.

The claimed reconstituted surfactants will be prepared by mixing solutions in organic solvents of the lipid mixture with the peptide or the protein and subsequently drying the preparation until a dry powder is obtained.

The invention also concerns pharmaceutical formulations consisting of a synthetic surfactant comprising a lipid mixture containing at least 10% by weight of polyunsaturated phospholipids for administration to subjects in need of a therapeutic treatment of surfactant deficiency.

Said pharmaceutical formulations will be administered intratracheally or by aerosol administration or by nebulisation in the form of suspensions in an aqueous medium or in a suitable propellant. Preferably they will be administered intratracheally as suspension in 0.9% normal saline. The surfactant concentration (expressed as lipid content) will be in the range of from about 2 to about 160 mg of surfactant per ml, preferably between 10 and 120 mg/ml, more preferably between 20 and 100 mg/ml, even more preferably between 40 and 80 mg/ml.

The surfactant of the invention will be used for the treatment of all cases of surfactant deficiencies and typically in the treatment of neonatal respiratory distress syndrome (RDS), acute respiratory distress syndrome in adults (ARDS), meconium aspiration syndrome (MAS), several types of pneumonia and bronchopulmonary dysplasia.

The advantages of the present invention will be illustrate by the following examples.

### Example 1 -Preparation of the reconstituted surfactants

### Materials

The phospholipids 1,2-dipalmitoyl-*sn*-glycero-3-phosphocholine (DPPC), 1,2-dipalmitoyl-*sn*-glycero-3-phosphoglycerol (DPPG), 1-palmitoyl-2-oleyl-*sn*-glycero-3-phosphoglycerol (POPG), 1-palmitoyl-2-linoleyl-*sn*-glycero-3-phosphocholine (PLPC) and 1-stearoyl-2-arachidonoyl-*sn*-glycero-3-phosphocholine (SAPC) have been used for preparing the lipid mixtures and have been commercially acquired.

The peptide SP-C33 was synthesised according to the method reported in WO 00/47623.

### Preparation of the reconstituted surfactants

The lipids, dissolved in chloroform/methanol 98:2 (v/v), were mixed in the proportions DPPC:PLPC:SAPC:DPPG:POPG in the ratio 45:30:10:5:10 by weight and DPPC:POPG in the ratio 68:31 by weight in order to obtain respectively a mixture according to the teaching of the present invention (lipid mixture A) and one according to the prior art (lipid mixture **B**). Corresponding reconstituted surfactant preparations (surfactant **A** and surfactant **B**) were prepared by adding SP-C33 to each of the lipid mixtures, at the amount of 0.02 by weight. The mixtures were evaporated under nitrogen and resuspended in 150 mmol/l NaCl at lipid concentrations of 10 mg/ml and 80 mg/ml, respectively.

A surfactant **C** according to the present invention was also prepared having the following composition: DPPC:liver PL: POPG=50:40:10 (80 mg/ml) with SP-C 33 (2%).

The phospholipid composition of the liver extract of the surfactant **C** is characterised as follows: phosphatidylcholine (PC) 50%, phosphatidyletanolamine (PE) 25%, sphingomyelin (SM) 10%, acidic phospholipids 15%.

### Example 2 -In vitro activity

Surface properties were determined in captive bubble surfactometer. The chamber was filled with a sucrose solution and 2 µl of surfactant **A** or surfactant **B** (10 mg/ml) was injected; a bubble was then created insufflating 10 microl of air. Surface tension at the air-liquid interface was determined from the shape of the bubble during film adsorption and subsequent quasi-static cyclic area compression.

Surfactant **A** demonstrated a better performance than the surfactant **B** in terms of less surface area compression required to reach minimal surface tension.

### Example 3 -In vivo activity of surfactant A

Pre-term rabbit fetuses (n = 34) were delivered at a gestational age of 27 days by caesarian section (term=31 days). At delivery, the animals were anaesthetized with intraperitoneal sodium pentobarbital (0.1 ml; 6 mg/ml), tracheotomized, paralyzed with intra-peritoneal pancuronium bromide (0.1-0.15 ml; 0.2 mg/ml) and kept in plethysmograph system at 37°C. They were mechanically ventilated in parallel with a modified Servo-Ventilator delivering 100% oxygen. After surfactant instillation, peak pressure was first raised to 35 cmH₂O for 1 min, to facilitate distribution of surfactant in the lungs, and then lowered to 25 cmH₂O. The animals were then ventilated with a peak pressure of 25 cmH₂O for 15 min, where after pressure was lowered to 20 cmH₂O for 5 min, and further on to 15 cmH₂O for 5 min, and then raised again to 25 cmH₂O for 5 min. Tidal volumes were recorded at 5 min intervals by means of a pneumotachograph connected to the pletysmograph box.

The frequency was 40 per minute, the inspiration:expiration time ratio 1:1. No positive end-expiratory pressure was applied.

The immature newborn rabbits were randomized to receive at birth, via the tracheal cannula, 2.5 ml/kg body weight of surfactant **A** or surfactant **B** (80 mg/ml). In control animals, no material was instilled into the airways. All animals were ventilated for 30 min.

As soon as arrhythmia, bradycardia (heart rate < 60/min), absence of QRS complexes or pneumothorax occurred, animals were sacrificed by intracranial injection of lidocaine (0.5 ml; 20 mg/ml).

Tidal volumes (V_{T}) were measured with a plethysmograph system as described in Sun et al, *Eur Respir J* 1991, 4, 364-370 and end-expiratory lung gas volumes (LGV) were estimated from the difference between lung volume and lung wet weight (Scherle et al *Mikroscopie* 1970, 26, 57).

In these experiments it was observed that surfactant **A** exhibits tidal volumes higher than those obtained with surfactant B. Also the Lung Gas Volumes values were remarkably increased.

### Example 4 -In vivo activity of surfactant C

The surfactant **C** (80 mg/ml) and a commercially available modified natural surfactant (Curosurf) (80 mg/ml) were compared for tidal volume and lung gas volume in the same animal model of Example 3: the reconstituted surfactant **C** prepared according to the invention showed an activity comparable to that of the modified natural surfactant.

## Claims

1. A lipid mixture comprising 1,2-dipalmitoyl-sn-glycero-3-phosphocholine (DPPC) in amount of less than 60% by weight of total phospholipids, and at least 5% by weight of polyunsaturated phospholipids of total phospholipids.

2. A lipid mixture according to claim 1 wherein DPPC is present in amount of about 40% to about 60% by weight.

3. A lipid mixture according to claim 2 wherein DPPC is present in amount of about 40% to about 55% by weight.

4. A lipid mixture according to claims 1 to 3 wherein the polyunsaturated phospholipids are present in amount of at least 10%.

5. A lipid mixture according to claims 1 to 4 wherein the polyunsaturated phospholipids are present in amount of at least 20%.

6. A lipid mixture according to claims 1 to 5 wherein the polyunsaturated phospholipids are present in amount of at least 30%.

7. A lipid mixture according to claims 1 to 6 wherein the polyunsaturated phospholipids are obtained by isolation from a natural source.

8. A lipid mixture according to claims 1 to 7 wherein the polyunsaturated phospholipids or DPPC or both are obtained by isolation from liver.

9. A lipid mixture according to claims 1 to 8 wherein the polyunsaturated phospholipids comprise phosphatidylethanolamine in amount of at least 1% by weight.

10. A lipid mixture according to claims 1 to 9 wherein the polyunsaturated phospholipids comprise phosphatidylethanolamine in amount of at least 5% by weight.

11. A lipid mixture according to claims 1 to 10 wherein the unsaturated phospholipids comprise acidic phospholipids.

12. A lipid mixture according to claims 1 to 11 wherein the unsaturated phospholipids comprise acidic phospholipids in amount of at least 5% by weight.

13. A lipid mixture according to claims 1 to 12 wherein the phospholipids comprise sphingomyelin in amount of at least 2% by weight.

14. A lipid mixture according to claims 1 to 13 devoid of palmitic acid.

15. A lipid mixture according to claims 1 to 13 in combination with one or more surfactant proteins selected from SP-A, SP-B, SP-C, SP-D, their synthetic or recombinant analogues.

16. A lipid mixture according to claim 15 wherein the surfactant protein is present in amount of about 0.1% to 20% by weight of total mixture.

17. A lipid mixture according to claims 14 to 16 wherein the surfactant protein is present in amount of about 1% to 10% by weight of total mixture.

18. A lipid mixture according to claims 15 to 17 wherein the surfactant protein is a synthetic or recombinant analogue of SP-C of general formula (**I**), according to the amino acids one-letter code:
FₑG_{f}IPZZPVHLKR(XₐB)(X_{b}B)ₙ(X_{c}B)ₘX_{d}GALLΩGL (**I**)
**characterised in that**:
X is an amino acid selected from the group consisting of I, L, nL (norleucine);
B is an amino acid selected from the group consisting of K, W, F, Y, ornithine;
Z is S optionally substituted with acyl groups containing 12-22 carbon atoms linked to the side chain via an ester or thio-ester bond, respectively;
Ω is an amino acid selected from the group consisting of M, I, L, nL;
a is an integer from 1 to 19;
b is an integer from 1 to 19;
c is an integer from 1 to 21;
d is an integer from 0 to 20;
e is 0 or 1;
f is 0 or 1;
n is 0 or 1;
m is 0 or 1;
with the following provisos:
- n+m ≥ 0;
- f>e,
- (XaB)n(XbB)n(XcB)mXd is a sequence having a maximum of 22 amino acids, preferably from 10 to 22.

19. A lipid mixture according to claims 15 to 18 wherein the SP-C analogue has the formula (**I**I):
IPSSPVHLKRLKLLLLLLLLILLLILGALLΩGL (**II**)
wherein Ω is an amino acid selected from the group consisting of M, I, L, nL.

20. A lipid mixture according to claim 6 wherein the SP-C analogue has the following amino acid composition:
IPSSPVHLKRLKLLLLLLLLILLLILGALLMGL
